# EUROPEAN PATENT APPLICATION

(11) **EP 4 675 630 A1**
(43) Date of publication of application: **07.01.2026**
(21) Application number: 24186786.0
(22) Date of filing: 05.07.2024
(51) Int. Cl.: G16B 40/20, G16H 50/20, C12Q 1/6886

(54) **IMPROVED CANCER DETECTION**

(71) Applicant: Cambridge Enterprise, Ltd., Cambridge Cambridgeshire CB2 1TN (GB)
(72) Inventor: FITZGERALD, Rebecca C., Cambridge, CB2 1TN (GB); KILLCOYNE, Sarah, Cambridge, CB2 1TN (GB); YUSUF, Aisha, Cambridge, CB2 1TN (GB)
(74) Representative: HGF

(57) **Abstract**

The invention relates to a computer-implemented method for detecting oesophageal squamous cell dysplasia (OSCD) or oesophageal squamous cell cancer (OSCC) in a cell sample from a subject. This invention also relates to a computer-implemented method for generating a trained model to detect OSCD or OSCC. This invention also relates to a computer-readable storage medium or a computer program comprising instructions which when executed by a computer, are capable of causing the computer to perform the method. This invention also relates to a kit comprising the computer program or computer-readable storage medium and a non-endoscopic cell collection device. The invention also relates to an apparatus for performing the method. The invention further relates to the use of genome wide instability scores (GWIS) as a diagnostic marker for cancer, wherein GWIS is a measure of the CNV across a plurality of autosomal arms in a cell sample from a subject.

## Description

### Field of the Invention

The invention relates to a computer-implemented method for diagnosing oesophageal squamous cell dysplasia or oesophageal squamous cell cancer in a patient and generating a model to perform the diagnosis. Also provided is a computer program storage medium or computer program which, when executed by a computer, cause the computer to carry out the methods and apparatus comprising means for carrying out the methods. The invention also relates to a novel biomarker for cancer diagnosis in a cell sample: GWIS.

### Background

Early detection of cancer can lead to hugely improved outcomes for patients. Outcomes for some cancers including oesophageal cancer have changed little despite the advances made in treating cancer with only 12% of oesophageal cancer patients surviving for ten or more years. However early detection poses the risk of overtreatment. Therefore, accurate biomarkers of early cancer progression are needed. Other ways of accurate detection other than by endoscopic procedures and pathology assessments are also needed so as to make detection faster.

### Summary of the Invention

The inventors have solved this problem by providing early detection methods for oesophageal squamous cell dysplasia which puts a patient at high risk of cancer. The methods disclosed herein also allows the accurate detection of dysplasia and oesophageal squamous cell cancer without the need for endoscopy and/or pathology assessment.

In a first aspect of the invention there is provided a computer-implemented method for detecting oesophageal squamous cell dysplasia (OSCD) or oesophageal squamous cell cancer (OSCC) in a cell sample from a subject, the method comprising:
a) obtaining the copy number variation (CNV) in the cell sample across chromosomal arms comprising: 2q, 3q, 9p and 11q;
b) inputting the CNV from step a) into a trained machine learning model which relates CNV across the chromosomal arms comprising 2p, 3q, 9p and 11q with OSCD or OSCC; and
c) outputting from the trained model a prediction comprising the presence or absence of OSCD or OSCC.

In a further aspect of the invention there is provided a computer-readable storage medium or a computer program comprising instructions which, when executed by a computer, cause the computer to carry out the above method.

In a further aspect of the invention there is provided a computer-implemented trained model obtained from the above method.

In a further aspect of the invention there is provided a kit comprising the above computer program or computer-readable storage medium and a non-endoscopic cell collection device In a further aspect of the invention there is provided an apparatus comprising:
a) a memory storing a trained machine learning model; and
b) processing circuitry communicatively coupled to the memory, the processing circuitry to run the trained machine learning model to receive as input CNV measurements from a cell sample from a subject, wherein the CNV measurements are across chromosomal arms comprising: 2q, 3q, 9p, and 11q; and
output a prediction corresponding to the presence of absence of OSCD or OSCC.

In a further aspect of the invention there is provided an apparatus comprising:
a) a memory storing a machine learning algorithm; and
b) processing circuitry communicatively coupled to the memory, the processing circuitry to run the machine learning algorithm to process training data to generate a trained machine learning model by:
   adjusting the parameter values of the machine learning algorithm to reduce an error function based on training data comprising CNV measurements and associated ground truth data,
   wherein the CNV measurements are across chromosomal arms comprising: 2q, 3q, 9p, and 11q in cell samples from each of a plurality of subjects;
   and wherein the associated ground truth data comprises, for each of the plurality of training subjects an indication of the presence or absence of OSCD or OSCC.

In a further aspect of the invention there is provided use of GWIS as a diagnostic marker for cancer, wherein GWIS is a measure of the CNV across a plurality of autosomal arms in a cell sample from a subject.

### Detailed description

### General terms

### Oesophageal squamous cell cancer (OSCC) and dysplasia (OSCD)

OSCC is one of the main types of oesophageal cancer. Oesophageal squamous cell dysplasia (OSCD) is altered oesophageal squamous epithelium with an increased likelihood for progression to OSCC.

Dysplasia is an abnormal condition in which cells may have altered shape or may divide in a manner that alters the appearance of the tissue or organ.

The degree of change ranges from minor to significant changes (low-grade - LGD), to serious or very abnormal changes (high-grade) dysplasia (HGD).

The diagnosis may be the presence of absence of HGD or OSCC.

### Sample

The sample is an oesophageal sample. The sample may be an endoscopic sample. That is a biopsy taken via an endoscope. The endoscopic sample may be taken from the upper oesophagus.

Alternatively, the sample may be a non-biopsy (non-endoscopic sample). For example the sample may be collected using a non-endoscopic cell collection device. For example, the sample may be collected via use of a swallowable cell collection device comprising an abrasive material configured to collect cells from the surface of the oesophagus. By abrasive is meant a surface rough enough to collect cells.

The cells may be collected by introducing a swallowable cell collection device into the subject, retrieving the device by withdrawal of the device through the oesophagus and collecting the cells from the device.

The abrasive material may be retained within a structure prior to release at the appropriate site of collection. The structure may be a soluble capsule or an inflatable balloon. By soluble is meant dissolvable to allow the abrasive material to be released for collection of cells. The device may have a means for retrieving or withdrawing the device from the oesophagus. For example the device (the abrasive material) may be attached to a rope, for example a string or a catheter. Cytosponge^{™} and EndoSign^{®} are examples of swallowable cell sampling devices. Also described is a kit comprising a non-endoscopic device as disclosed above, and a computer program or computer-readable storage as described below.

DNA from the collected cell sample is extracted and subject to any of the techniques described below to ascertain CNV.

### CNV (copy number variation) for OSCD and OSCC

CNV may be measured in the DNA from the collected sample by any means known in the art. For example by performing whole genome sequencing, for example shallow WGS (for example, up to 2x depth, 3x depth, 4x depth, or for example wherein the sequencing depth is 5 or less, 10 or less). CNV may then be determined by analyses of the sequencing data. Other methods of measuring copy number may also be used. For example, exome or methylation sequencing; or SNP arrays. The CNV may therefore be determined by sequencing. Optionally, SNP arrays are not used for the measurement of CNV.

Extracting CNV from the data (e.g. whole genome sequencing data) may be done by any means known in the art. The CNV may be calculated from sequencing data by dividing each arm into bins and counting the reads within each bin. A measurement of the total CNV across the arm is then taken from these counted bins. For example, the average or median count for the bins across an arm may be taken. This value may then be log2-transformed.

CNV is determined across the one or more chromosomal arms. That is, the entire chromosomal arm is analysed to determine the CNV for that arm. Aberrant CNV in the following arms is related to diagnosis of oesophageal squamous cell dysplasia or oesophageal squamous cell cancer:
2q, 3q, 9p and 11q.

Software packages for calculating CNV are provided in the examples. For example QDNAseq. QDNAseq identifies copy number alterations (CNAs) in sequencing data, even with low depth, without a reference sample. It estimates copy number using depth of coverage, dividing the genome into bins and counting reads in each bin to infer copy number without a reference signal.

### Additional inputs for the trained model

As well as the CNV of the chromosomal arms listed above, the trained model can also be trained to relate additional inputs with detection of OSCD or OSCC. These additional inputs variables can be:
- a genome wide instability score; and/or
- cytological evaluation; and/or
- immunohistochemistry data.

### Genome wide instability score

The Genome-Wide Instability Score (GWIS) is a quantitative metric to measure genomic instability.

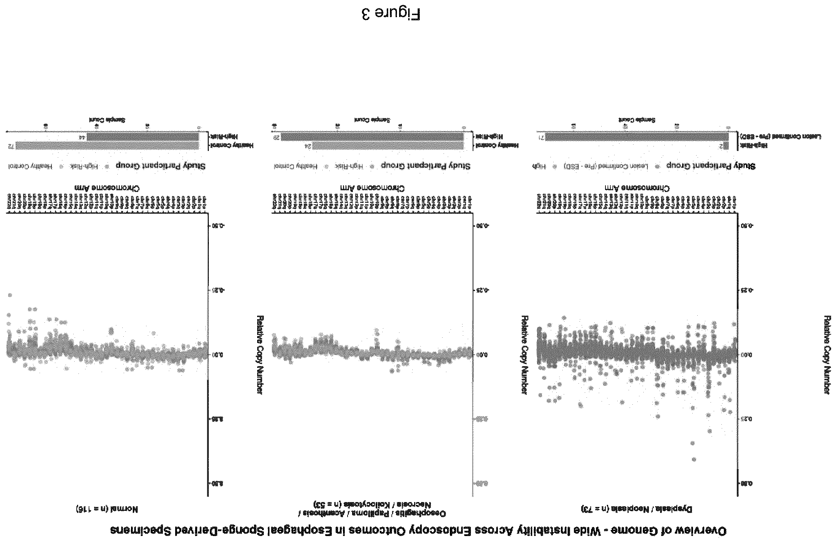

By genome-wide is meant copy number variation is analysed from a plurality of chromosomal arms from the autosomes. The sex chromosomes may not be included in the analysis. For example, at least 20 or at least 30 chromosomal arms may be analysed for the calculation of GWIS. For example, the chromosomal arms used may be those listed in Figure 3, i.e. Chr1p, Chr1q, Chr2p, Chr2q, Chr3p, Chr3q, Chr4p, Chr4q, Chr5p, Chr5q, Chr6p, Chr6q, Chr7p, Chr7q, Chr8p, Chr8q, Chr9p, Chr9q, Chr10p, Chr10q, Chr11p, Chr11q, Chr12p, Chr12q, Chr13q, Chr14q, Chr15q, Chr16p, Chr16q, Chr17p, Chr17q, Chr18p, Chr18q, Chr19p, Chr19q, Chr20p, Chr20q, Chr21q and Chr22q. That is, the analyses may consist of a score from at least 20 or at least 30 chromosomal arms (autosomal arms), for example the above autosomal arms.

The genome wide instability score (GWIS) may be calculated from whole genome sequencing data (e.g. shallow WGS). This data may be processed as follows:
- a reference genome is divided into segments (i.e. the genome is divided into a plurality of bins);
- reads from the sequencing are allocated to the bins; and
- the number of reads in each bin is calculated.

Windows which have significant copy number alterations (compared to the same windows as calculated for a control) may be identified and counted, and then the sum is divided by the total number of windows across the genome. This results in a percentage score indicating the level of genomic instability.

For example only, if after binning there are 60 bins which are significantly altered in terms of their CNV, then the GWIS would be 60/total number of windows. Essentially, the GWIS is a percentage indication of error compared with a reference genome. The higher the GWIS, then the more alterations have happened in the genome meaning the higher chance of abnormal cell changes.

The genomic window may be 50kb. The genomic window may be from 50kb to 200kb.

To identify windows with significant CNV, the threshold may be determined, for example by using the Tukey outlier formula, to identify outliers within a dataset, for example based on the interquartile range (IQR). The median and interquartile range (IQR) may be utilized for threshold calculation: thresholds may be set at median ±1.5 IQR to identify moderate outliers. That is, the threshold is determined based on the variation in the control group and variation outside the control group, the latter indicative of significant alteration.

In the context of genomic data analysis, the threshold is applied within specific segments (e.g., chromosomal arms). That is, a threshold is identified for each arm, above which the bin is indicated as having an altered number of CNV compared to the reference. By conducting outlier detection within each chromosomal arm's context, genomic complexity scores are calculated that accurately capture regional variability having accounted for variations in baseline copy number values across different arms. Rather than applying a uniform threshold, this approach recognizes the diverse nature of copy number alterations across genomic regions.

After assigning reads to each bin, the reads may be counted and this total may be log2 transformed prior to identifying the bins with significant copy number alteration.

The GWIS may also be used as a general digital biomarker, i.e. useful to aid diagnosis of other cancers.

### Cytological evaluation

The cytological evaluation data may include categorisation of cells as any of: atypical squamous cells of undetermined significance (AUS), low-grade dysplasia (LGD), high-grade dysplasia (HGD), and oesophageal squamous cell carcinoma (OSCC) or normal findings (normal squamous oesophagus or inflamed oesophagus called oesophagitis, i.e. non-pathogenic).

### Immunohistochemistry data

The data may also include immunohistochemistry. That is, the cell sample may be tested (e.g. stained) for the presence of a biomarker, e.g. p53.

### Model

By model is meant an algorithm or set of algorithms which relate the CNV to the presence of absence of OSCD or OSCC.

The model is a machine learning model. The model may be a trained logistic regression model. For example, a penalised logistic regression with elastic net regularisation.

Data is used to train an algorithm to identify samples from individuals with dysplasia or cancer. The regression algorithm may be followed by a classification algorithm to allocate the subject to, e.g. presence or absence of dysplasia or cancer.

### Output

The output of the trained diagnostic model is the presence of cancer or dysplasia or not.

The model may generate predictive probability scores for each sample, indicating the likelihood of dysplasia or cancer. Samples with predictive probability scores surpassing a threshold, determined from the analysis conducted during modelling, are considered positive for dysplasia or cancer; or at risk of progression.

### Method steps for training the models:

### Training data:

CNV is calculated from DNA samples from a subject as described above. The CNV measurements from arms 2q, 3q, 9p and 11q are used as input variables. Samples are labelled as having dysplasia, or as being cancerous or non-cancerous. That is, samples from patients with oesophageal squamous cell dysplasia or oesophageal squamous cell cancer; and samples from subjects without these conditions are used.

Additional input features may be: GWIS and/or cytological evaluation; and or immunohistochemistry data as described above.

### Training the model:

The model is trained to relate the data to the input variables to the labelled outcomes: cancerous, non-cancerous or presence of dysplasia.

To optimise hyperparameters, a cross-validation approach may be used. This may also be used to evaluate the performance of the model across a range of alpha values. The cross-validation approach may be a k-fold cross-validation approach.

### Method of using the trained model

*Input:* Once trained, data for a new patient can be input into the trained model. That is, CNV measurements and optionally one or more of the further input variables as described above.

*Output:* The output from the trained model is an indication of the presence or absence of OSCD or OSCC.

The trained model may be used to identify patients with no previous history of OSCD/OSCC. The trained model may be used to monitor OSCD patients to check for progression of OSCD to OSCC. Alternatively, the trained model may also be used post-treatment of OSCC to monitor for any relapse.

### Computer program and non-transitory media

By computer program is meant machine readable program instructions. These may be provided on a transitory medium such as a transmission medium or on a non-transitory medium such as a storage medium. Such machine readable instructions (computer program code) may be implemented in a high level procedural or object oriented programming language. However, the program(s) may be implemented in assembly or machine language, if desired. In any case, the language may be a compiled or interpreted language, and combined with hardware implementations. Program instructions may be executed on a single processor or on two or more processors in a distributed manner.

Therefore also included are one or more non-transitory computer readable media storing machine-readable instructions which, when executed, cause one or more processors to perform the method described herein.

### Data processing apparatus

The processing circuitry of the apparatus may be communicatively coupled to a memory. The processing circuitry may comprise general purpose processor circuitry configured by program code to perform specified processing functions. Alternatively, the processing circuitry may comprise special purpose processing circuitry. Thus, the configuration of the circuitry to perform its specified function may be limited exclusively to hardware, limited exclusively to software, or a combination of hardware modification and software execution.

Additional input variables or inputs for the data apparatuses are as described above with reference to the methods.

Throughout the specification, unless the context demands otherwise, the terms 'comprise' or 'include', or variations such as 'comprises' or 'comprising', 'includes' or 'including' will be understood to imply the method or kit includes a stated integer or group of integers, but not the exclusion of any other integer or group of integers.

Each document, reference, patent application or patent cited in this text is expressly incorporated herein in their entirety by reference, which means it should be read and considered by the reader as part of this text. That the document, reference, patent application or patent cited in the text is not repeated in this text is merely for reasons of conciseness. Reference to cited material or information contained in the text should not be understood as a concession that the material or information was part of the common general knowledge or was known in any country.

### Description of the Figures

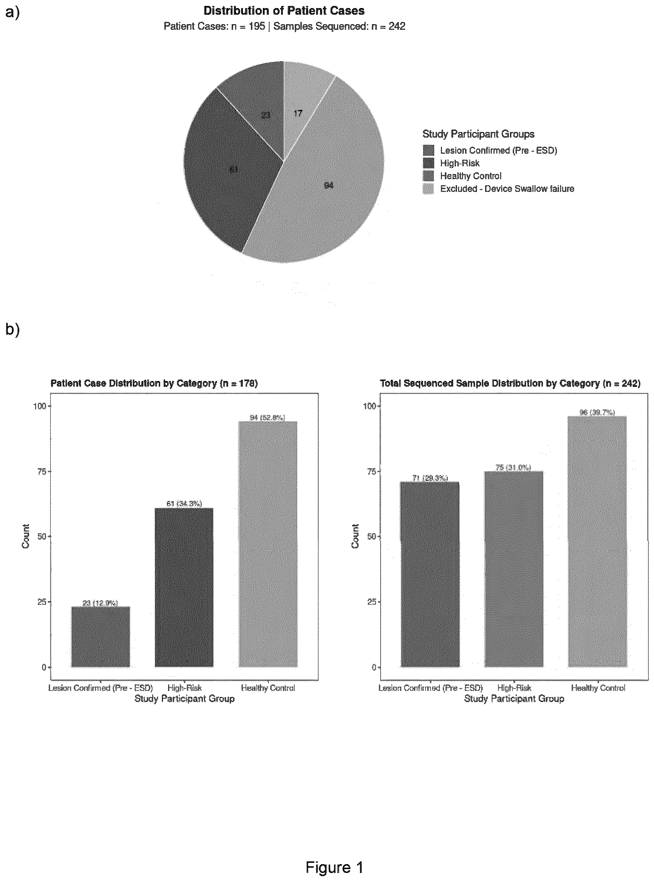

**Figure 1a** **shows the distribution of patient cases in the EDEN study.** Figure 1b shows the case distribution by category and the total sequenced sample distribution by category.

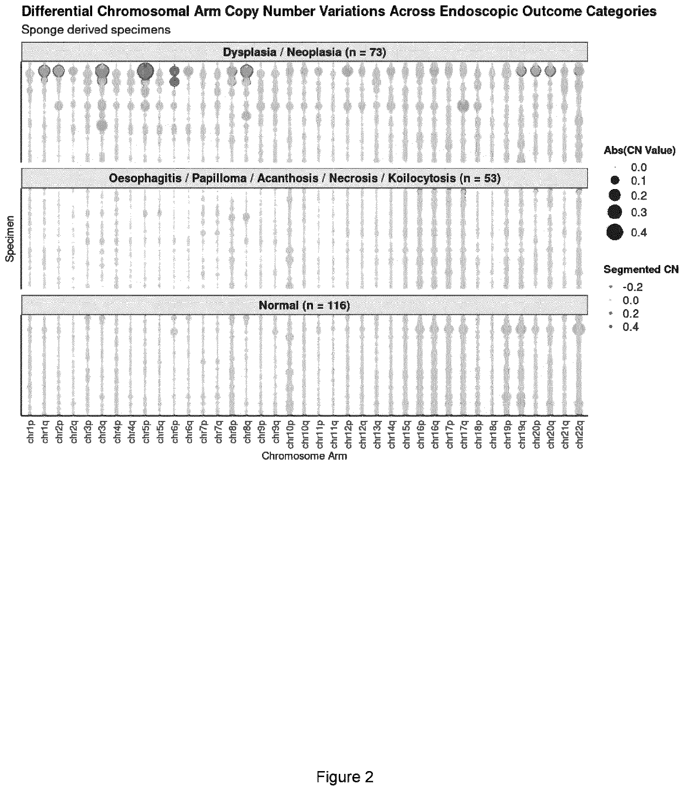

**Figure 2** **shows differential chromosomal arm copy number variations across endoscopic outcome categories for sponge derived specimens.** This figure provides a stratified visualization of chromosome arm copy number alterations within the three sponge sample groups based on endoscopy outcome. Each bubble's dimension is proportional to the absolute value of the copy number variation (CNV) observed at specific loci across the chromosome arms, serving as a metric of genomic alteration. The colour coding-ranging from blue for losses to red for gains-reflects the nature and degree of these variations, with more intense hues signifying greater deviations from normal copy numbers.
**Figure 3** **shows an overview of Genome Wide Instability across endoscopy outcomes in oesophageal sponge-derived specimens.** The scatter plot shows raw relative log2 copy number values of sponge samples across the three endoscopy outcome groups. The plot revealed the dysplasia/neoplasia group to harbour significant genome wide instability.

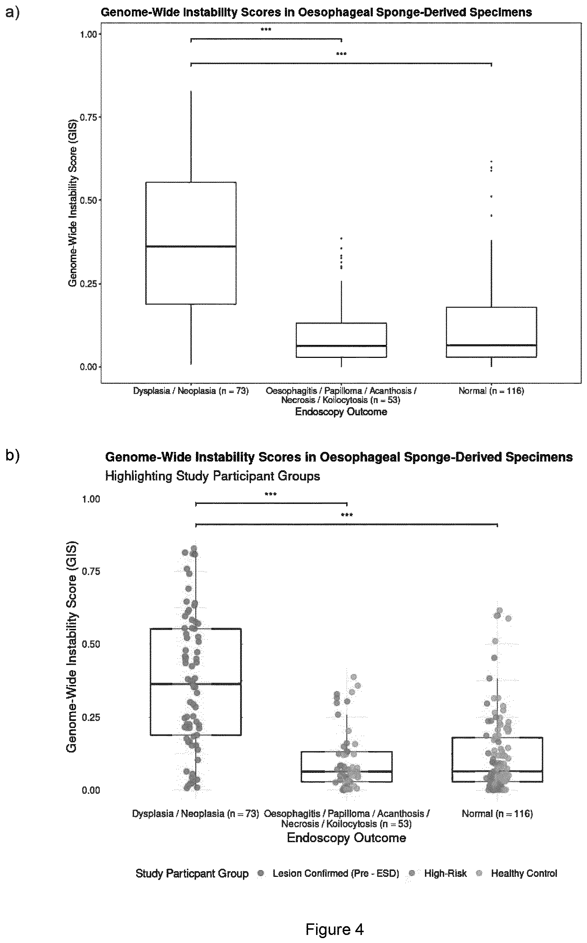

**Figure 4a** **shows Genome Wide Instability scores in oesophageal sponge derived specimens.** Figure 4b highlight study participant groups. Box plots show the genome wide instability score (GWIS -a composite measure derived from prior assessments, quantifying the extent of genomic disruption across the entire genome). These scores are plotted for each sponge sample belonging to the three endoscopy outcome groups - coloured by study participant group.

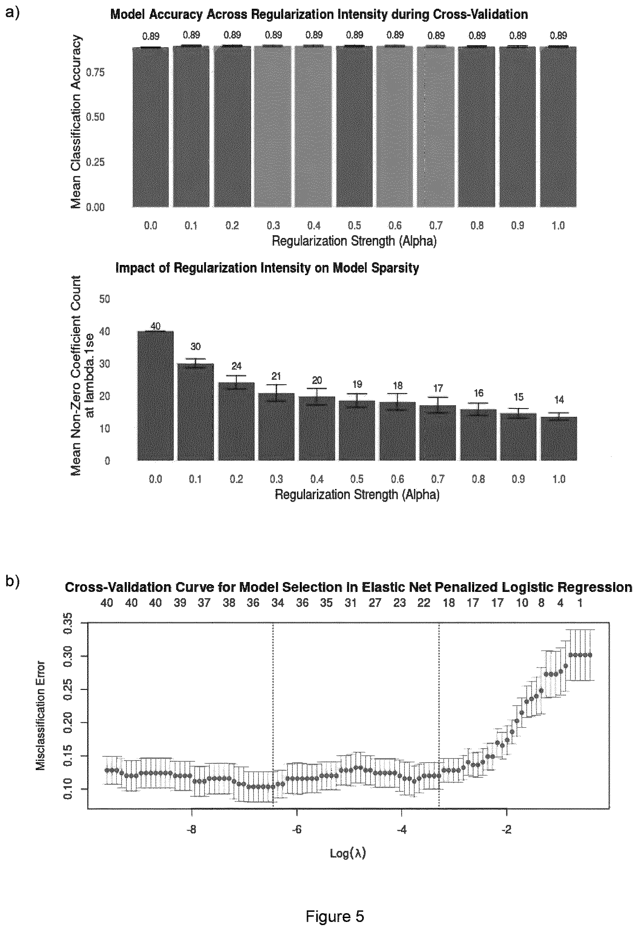

**Figure 5a** **shows model accuracy across regularization intensity during cross-validation and the impact of regularization intensity on model sparsity.** The red rectangle shows the tuning parameter used for the final model selected with mean classification accuracy. Figure 5b shows the cross-validation curve for model selection in elastic net penalized logistic regression.

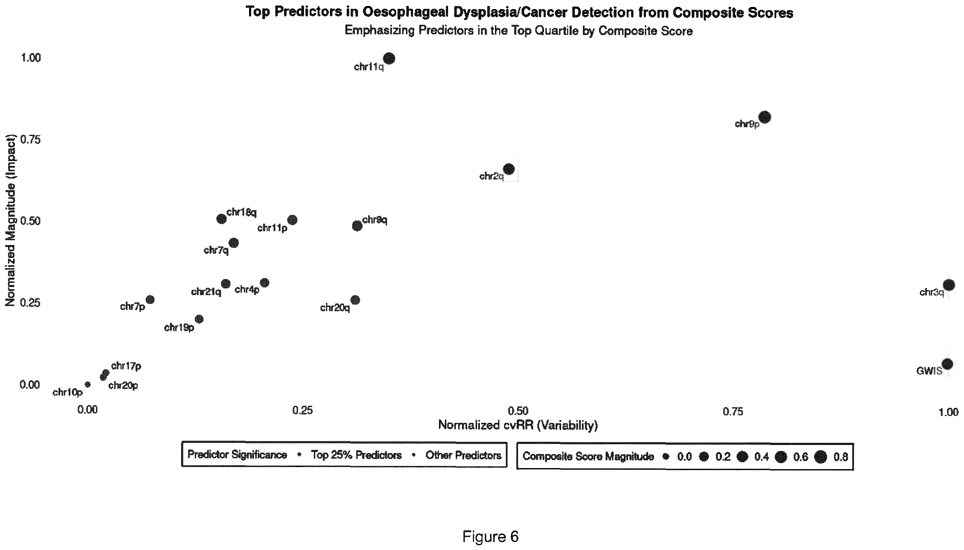

**Figure 6** **shows the top predictors in oesophageal dysplasia/ cancer detection from composite scores.**

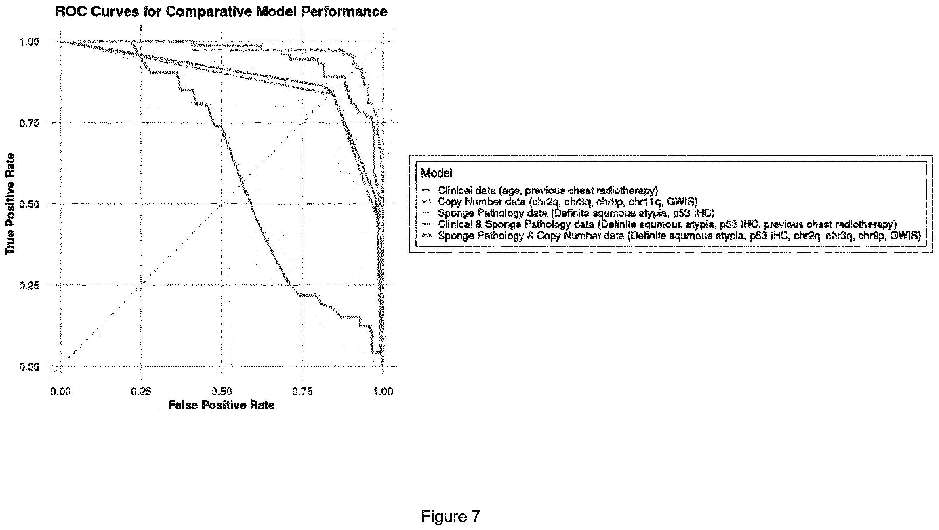

**Figure 7** **shows the ROC curve for comparative model performance.**

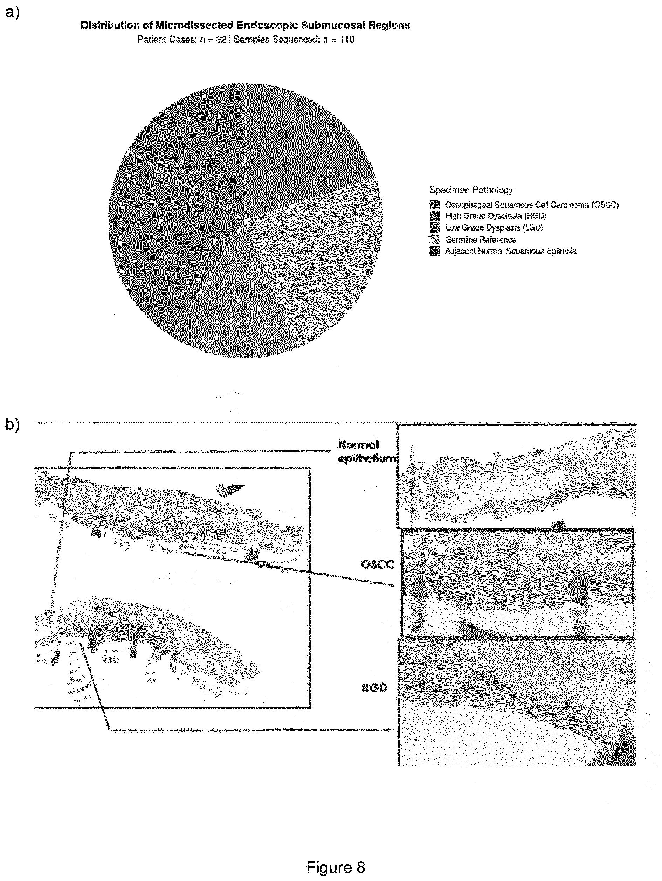

**Figure 8a** **shows the distribution of microdissected endoscopic submucosal regions in the validation study.** Figure 8b shows an example of the multi-region endoscopic submucosal dissection specimens.

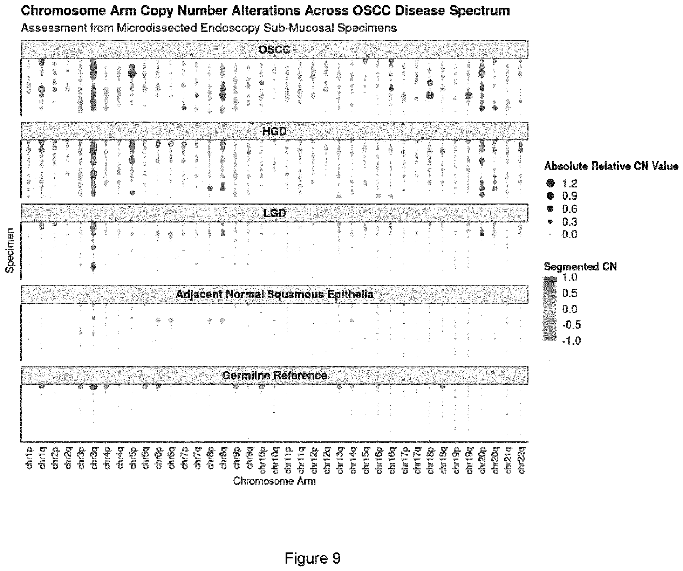

**Figure 9** **shows chromosome arm copy number alterations across OSCC disease spectrum from microdissected endoscopy sub-mucosal specimens.** This figure provides a stratified visualization of chromosome arm copy number alterations within the continuum of Oesophageal Squamous Cell Carcinoma (OSCC) development and its antecedent conditions. Each bubble's dimension is proportional to the absolute value of the copy number variation (CNV) observed at specific loci across the chromosome arms, serving as a metric of genomic alteration. The colour coding-ranging from blue for losses to red for gains-reflects the nature and degree of these variations, with more intense hues signifying greater deviations from normal copy numbers. The plot draws attention to recurrent genomic regions that may harbour oncogenic drivers or tumour suppressor genes

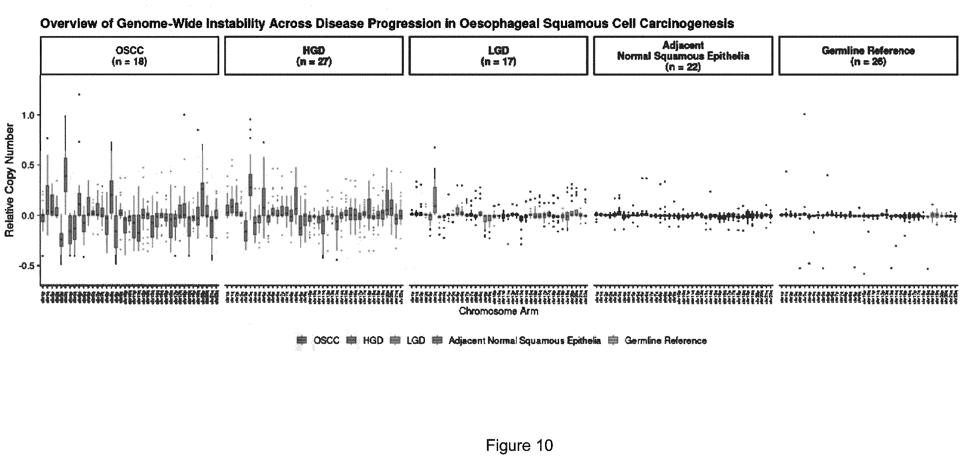

**Figure 10** **shows an overview of genome-wide instability across disease progression in oesophageal squamous cell carcinogenesis across each specimen pathology group.**

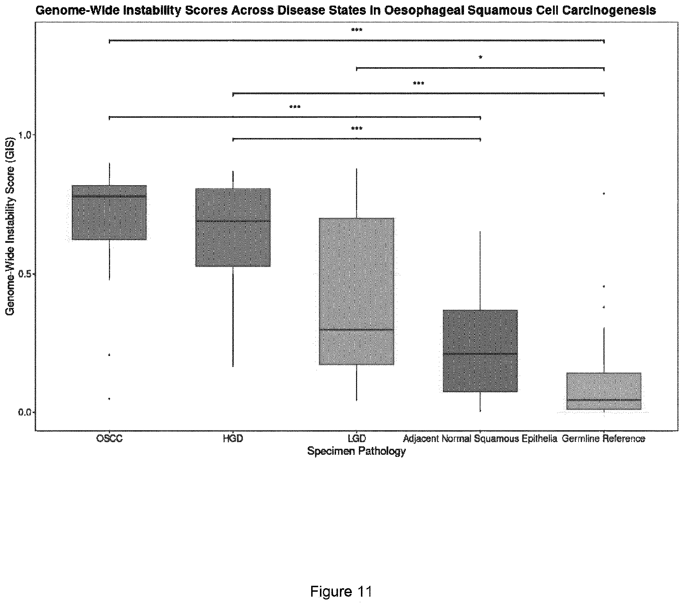

**Figure 11** **shows genome-wide instability score across disease states in oesophageal squamous cell carcinogenesis across each ESD specimen pathology group.**

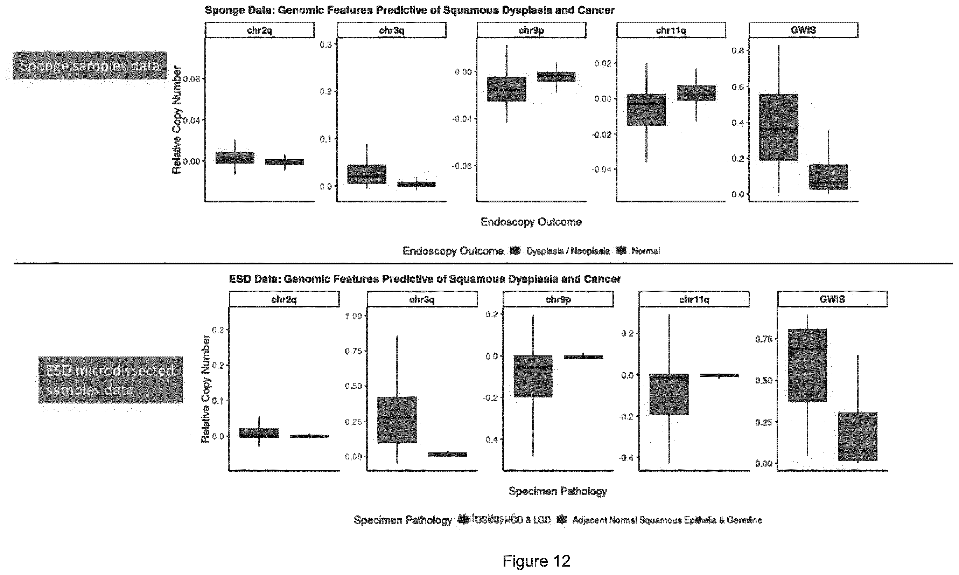

**Figure 12** **shows the genomic features identified in sponge data which are predictive of squamous dysplasia and cancer and recapitulated in ESD data.**

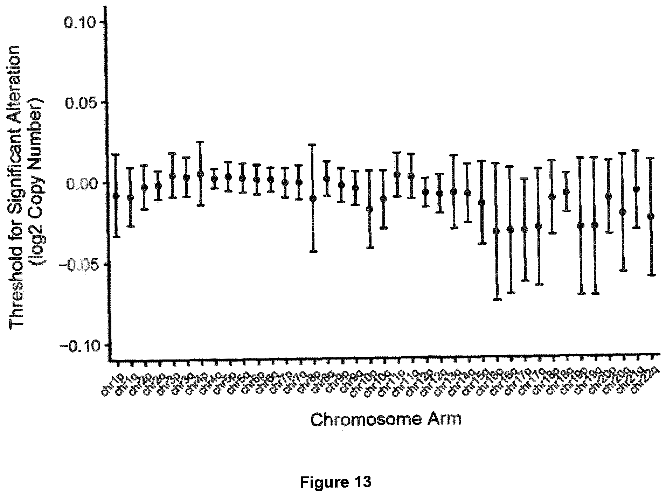

**Figure 13****: Determination of chromosome arm-specific thresholds for significantly altered 50kb genomic segments.** This error bar plot illustrates the Tukey outlier thresholds, which are represented by the median ± 1.5 times the interquartile range, for significant copy number alterations across each chromosomal arm. The plot indicates the median log₂-transformed relative copy number values (black dots) and the range of variability (error bars) calculated using healthy control sponge samples. These thresholds play a critical role in identifying genomic segments that surpass normal variation, thereby aiding accurate computation of the Genome-Wide Instability Score (GWIS). Customising these thresholds for each chromosomal arm enables a nuanced evaluation of genomic instability, thus enhancing the reliability of the metric.

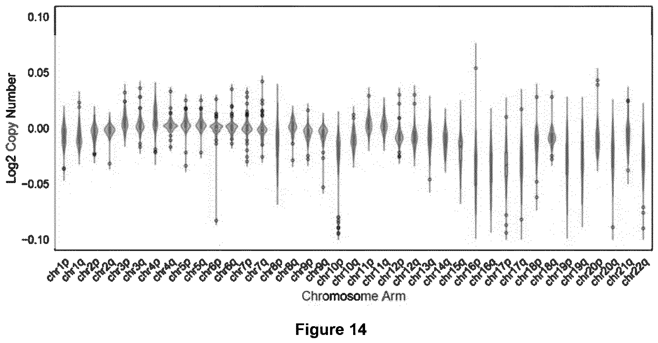

**Figure 14****: Variability of relative copy number values across chromosomal arms.** Each violin plot, filled with green colour, represents the distribution of log₂-transformed relative copy number values for a specific chromosomal arm in cytosponge samples from the healthy control group (n=96). The width of the violin indicates data density. Embedded within the violin plots are box plots, coloured grey, showing the median, interquartile range, and outliers of the log₂-transformed copy number values. This visualisation emphasises the importance of customising thresholds for significant copy number alterations on an arm-specific basis, as some chromosomal arms exhibit tightly clustered relative copy number distributions, indicating stability, whereas others show broader distributions, reflecting greater variability. This customisation allows the identification of genuine deviations that may indicate pathological changes.

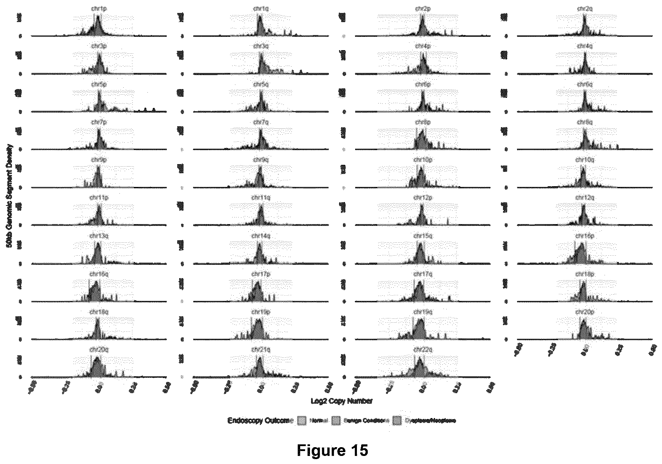

**Figure 15****: Chromosome arm-specific copy number profiles across endoscopic findings groups.** This plot illustrates the density of log₂-transformed relative copy number values across various chromosomal arms for different endoscopic findings groups: normal (n=17, in green), benign conditions including oesophagitis, papilloma, acanthosis, necrosis, or koilocytosis (n=17, in blue), and oesophageal squamous dysplasia/neoplasia (n=18, in red). Vertical blue and red lines indicate the lower and upper bounds of the significant alteration thresholds, respectively, as determined by the Tukey outlier method. These thresholds are crucial for identifying genomic segments that deviate significantly from normal variation, highlighting the potential areas of genomic instability relevant to disease progression. Each panel corresponds to a specific chromosomal arm, showing unique distribution patterns and thresholds pertinent to each genomic region. The y-axis is log-transformed using the natural logarithm plus one (log1p) to better visualise the density of genomic segments, particularly when values span several orders of magnitude.

### Examples

Aspects of the present invention will now be illustrated by way of example only and with reference to the following experimentation.

### Participants, data pre-processing and study objectives

### Participants

There were 195 number of patients included in the OSCAR and EDEN studies (Early Detection and surveillance of Oesophageal cancer with the Cytosponge^{™} and Nucleic acid biomarkers) as shown in Figure 1. The first group of patients consisted of control individuals presenting with dyspeptic symptoms. The second group included high risk-individuals comprising patients who had previously undergone curative treatment for histologically confirmed head and neck squamous cell carcinoma (HNSCC) of the larynx, oral cavity, oropharynx, or hypopharynx at least one year before enrolment. The third group consisted of patients with histologically proven oesophageal squamous dysplasia or neoplasia, undergoing evaluation for endoscopic treatment.

**Table 1: Demographics and clinical characteristics of study participants**

| **Characteristic** | **Control** | **High-Risk** | **Neoplasia** |
|---|---|---|---|
| **Age: mean (±SD) years** | 55.7 (±13.5) | 64.4 (±11.8) | 66.4 (±9.2) |
| **Sex: male; no. (%)** | 56 (53.3%) | 49 (69.0%) | 11 (45.8%) |
| **BMI: median cm/m² (IQR)** | 26.2 (22.9-28.7) | 26.4 (24.4-28.6) | 24.3 (22.4-28.6) |
| **Smoking: active and former; no (%)** | Active: 11 (10.9%) | Active: 9 (17.3%) | Active: 2 (8.7%), |
| **Lesion location: no. (%)** | N/A | N/A | Distal: 27.8%, |
| | | | Middle: 33.3%, |
| | | | Proximal: 38.9% |
| **Histopathology: no. (%)** | Normal: 80 (76.2%), | Normal: 41 (57.7%), | LGD: 2 (8.3%), |
| | | | HGD: 5 (20.8%), |
| | Benign conditions: 25 (23.8%) | Benign conditions: 29 (40.8%), | pTis OSCC: 3 (12.5%), |
| | | | pTla OSCC: 5 (20.8%), |
| | | HGD: 1 (1.4%) | pTlb OSCC: 7 (29.2%), |
| | | | T2 OSCC: 2 (8.3%) |

### Cytosponge sample processing

Endoscopic procedures were conducted by local gastroenterologists either on the same day as the Cytosponge procedure (OSCAR study) or within two months of it (EDEN study).

After retrieval, the anonymised Cytosponge specimens were processed to allow the cells to form a clot. The clots were subsequently processed into formalin-fixed paraffin-embedded (FFPE) blocks. The FFPE blocks were then sectioned into consecutively numbered sections and scrolls. Specifically, four scrolls were obtained from each set, which were reserved for DNA extraction and subsequent molecular analyses. Sections from the FFPE blocks were placed on regular and positively charged glass slides to facilitate various staining procedures. For cytological evaluation, haematoxylin and eosin (H&E) staining and p53 immunohistochemistry (IHC) were performed on prepared slides. Two expert cytopathologists blinded to the reference endoscopic diagnoses, conducted the evaluations. Squamous cell atypia was assessed in both superficial and deep sections processed for H&E staining. Similarly, p53 IHC (Novocastra NCL-L-p53-D07, Newcastle, UK; 1:50 dilution) was performed on adjacent superficial and deep FFPE slides using the BondMax autostainer with the Leica Bond Polymer Detection kit (Leica Biosystems, Milton Keynes, UK), following the manufacturer's instructions. Sections from the HET1A cell line were used as positive controls.

### Cytosponge sample cytological assessment

Cytological evaluation of the Cytosponge samples was conducted in accordance with the Bethesda System, a widely accepted standardised terminology for reporting cervical cytology. The cytological diagnoses were categorised as follows: normal, atypical squamous cells (ASC) indicating clear-cut dysplasia, and atypical squamous cells of undetermined significance (ASCUS). The ASCUS category was assigned to cellular abnormalities that were more pronounced than those attributable to reactive changes but did not meet the criteria for a definitive diagnosis of squamous dysplasia, either quantitatively or qualitatively. Specimens exhibiting cytological features suggestive of squamous dysplastic lesions were classified as ASC. p53 expression was assessed across all fields of the immunohistochemically stained slides. The results were scored based on the intensity of nuclear staining observed in squamous cells. Slides displaying one or more squamous cells with nuclear staining intensity comparable to that of the positive control slides were considered p53-positive. Slides lacking intense nuclear staining were scored as p53-negative. Notably, the absence of p53 staining cannot be reliably determined from Cytosponge samples, potentially leading to missed cases (another reason for the combined use with the chromosomal arm model which provides an improved analyses over standard cytological analysis of the cells obtained from the cytosponge collection device). Biomarker tests were considered positive if there was a consensus diagnosis from at least two expert pathologists. In some cases, the evaluation of p53 along with H&E staining helped clarify the diagnosis of atypia. To ensure accuracy, any sample with suspected atypia or aberrant p53 expression was reviewed by another pathologist following best practices for reporting dysplasia in biopsies.

### Stratified sampling strategy for biomarker evaluation

This study marked the first application of the shallow whole-genome sequencing (sWGS) assay to Cytosponge-derived specimens. Emphasis was placed on evaluating the performance of the assay and molecular biomarker (copy number alterations) across multiple sections in cases of dysplasia or neoplasia, which are the clinically significant conditions of interest. To achieve this, three sets of scrolls were derived from each of the 23 Cytosponge specimens in the neoplasia group, with each sample being run through the sWGS assay and analysed separately In the high-risk group, consisting of 61 specimens, two sets of scrolls were obtained from 14 randomly selected cases; each sample was also run through the sWGS assay and analysed separately. By sequencing 14 cases with two scrolls each, the total number of samples sequenced from the high-risk group (75) was brought closer to the total from the neoplasia group (71), ensuring a balanced representation across risk categories. The remaining 47 high-risk cases and 93/94 healthy control cases were processed with one scroll set each. This stratified sampling strategy enabled a comprehensive evaluation of the biomarker's performance, particularly in dysplastic/neoplastic cases, while also assessing its specificity in high-risk and healthy individuals.

### Cytosponge DNA library preparation

A total of 242 DNA samples were obtained from 178 Cytosponge tissue blocks across the three study groups (96 healthy controls, 75 high-risk, and 71 dysplasia/neoplasia) from the EDEN and OSCAR studies. In prior experiments, the NebNext Ultra II FS Kit (New England Biolabs, UK) was identified as optimal for whole-genome library preparation from fresh frozen cell line samples. This kit was further optimised for FFPE DNA using microdissected endoscopic submucosal dissection specimens.

For DNA extraction, four 10 µm sections were used per sample. The DNA extraction process employed the Deparaffinization Buffer and the GeneRead^{™} DNA FFPE Tissue Kit (Qiagen, Hilden, Germany), adhering to the manufacturer's protocol. To optimise the DNA yield, samples were incubated overnight at 56°C with proteinase K. Following this, the extraction was completed with a 10-minute incubation at room temperature, followed by a double elution in 15 µ! buffer ATE to ensure thorough DNA recovery.

Post-extraction, DNA quantification was performed using the Qubit^{®} dsDNA High Sensitivity Assay Kit on a Qubit^{®} 2.0 fluorometer, in accordance with the manufacturer's instructions (Thermo Fisher Scientific, Waltham, Massachusetts, USA). The DNA yields ranged from 2 ng/µl to 50 ng/µl, with an average yield of 19.17 ng/µl. DNA quality was assessed using the Agilent Genomic DNA ScreenTape system (Agilent Technologies, Santa Clara, USA). This system utilises automated electrophoresis to analyse genomic DNA integrity and size distribution ranging from 100 to over 60,000 base pairs (bp). The results predominantly showed DNA fragments exceeding 1,000 bp, indicating lower fragmentation than typically observed in FFPE samples. The samples had high DNA Integrity Number (DIN) scores, averaging 5.3, which suggests good quality and integrity suitable for subsequent genomic analyses.

The final NEBNext Ultra II FS Library Prep protocol, suitable for input DNA ≤ 100 ng, is summarised as follows. Input DNA was fragmented at 37°C for 5 min to achieve 300-350 bp fragments. The fragmented DNA underwent end-repair, 5'-phosphorylation, and terminal dA nucleotide addition in a single tube. The NEBNext Adaptor for Illumina (diluted 10-fold for inputs <50 ng) was ligated, followed by cleanup with 0.8X Agencourt AMPure XP beads. Adapter-ligated products were barcoded using NEBNext multiplex oligos for Illumina and amplified with seven PCR cycles for inputs ≥50 ng and nine cycles for inputs ≤50 ng. The libraries underwent a final cleanup with 0.9 × AMPure beads to remove primer dimers and other impurities.

### Cytosponge library quality control, quantification, pooling, and sequencing

Post-PCR amplification and cleanup, the quality of each library was assessed using the Agilent High Sensitivity D1000 ScreenTape System to verify DNA fragment size distribution and integrity, ensuring the absence of primer-dimer contamination. DNA library quantification was conducted using a Qubit dsDNA Broad Range (BR) Assay Kit on a Qubit 3.0 Fluorometer (Thermo Fisher Scientific, Waltham, Massachusetts, USA). Accurate quantification facilitated normalisation and ensured uniform library representation in the pooled sample, thus preventing biased sequencing results owing to library concentration disparities. Normalised libraries were pooled, targeting a sequencing coverage of 1x-2x per sample. This range was based on computational simulations and empirical data recommending a coverage depth of 1.5x-2x for reliable CNAs detection in high cellularity, microdissected FFPE samples. Sequencing was performed on the NovaSeq 6000 system using paired-end, 50 bp chemistry.

### Shallow whole genome sequencing pipeline

Shallow whole-genome sequencing was performed using single-end 50-bp reads on the Illumina HiSeq system, aiming for a sequencing depth of approximately 1.5 - 2x. Sequencing reads were aligned to the human reference genome (GRCh37) using Burrows-Wheeler Aligner (BWA) software, version 0.7.15. After alignment, reads were processed to adjust for mappability and GC content using the QDNAseq tool configured to a resolution of 50-kb bins. Following this preprocessing, only autosomal sequences were retained for further analysis, excluding any sequences with inadequate mappability or requiring significant GC correction. For the analysis of copy number abnormalities, the segmented data were processed using the piecewise constant fit method available in the 'copynumber' package of R Bioconductor, version 1.16. This analysis was based on the GC-adjusted read counts provided by the QDNAseq output, allowing for the precise identification of uniform copy number segments across the genome.

Shallow whole genome sequencing (sWGS) offers a cost-effective alternative to deep WGS while still providing better resolution and coverage than SNP arrays. By sequencing the genome at a lower depth, sWGS can detect CNVs typically down to 50-100 kilobases, making it suitable for studies where intermediate resolution is sufficient. The uniform genome-wide coverage of sWGS allows for the detection of CNVs in regions not covered by SNP arrays.

### QDNAseq data preprocessing

The analysis commenced with a preprocessing phase to manage and structure the high-resolution dataset generated by QDNAseq. This dataset encompassed log2-transformed relative copy number values for over 49,000 genomic segments, each spanning 50 kilobases (kb) across the entire genome. The initial step in data handling involved annotating each 50kb genomic segment with its corresponding chromosomal arm designation. Each 50kb segment was annotated with its corresponding chromosomal arm using the BEDTools software by intersecting the genomic bin coordinates with cytoband data from the UCSC Genome Browser, which provided detailed chromosomal arm designations. This annotation process ensured accurate mapping of each genomic segment to its respective chromosomal arm, which is a prerequisite for subsequent data manipulation and analysis at the chromosomal arm level. To streamline the analysis and enhance interpretability, log2-transformed copy number values were further aggregated into median values for each of the 39 autosomal chromosomal arms. This aggregation consolidated the data from over 49,000 genomic segments into 39 columns, each representing one of the autosomal chromosomal arms. This substantial reduction in data complexity facilitated subsequent analyses while preserving essential information crucial for exploring genomic instability patterns and identifying critical genomic alterations associated with OSCC progression.

### GWIS score calculation

The Genome-Wide Instability Score (GWIS) is a quantitative metric to measure genomic instability from sWGS data by analysing copy number values (e.g. log2-transformed) within genomic segments across each chromosomal arm.

To calculate the GWIS, a data-driven approach was used to establish robust and standardised thresholds for detecting significant copy number alterations. This process involves analysing log2-transformed relative copy number values from samples within the healthy control group to establish a baseline for normal variation, which is crucial for distinguishing genuine genomic deviations that indicate instability. To account for the inherent variability observed across the genome, thresholds for identifying significant changes were customised for each chromosome arm (Fig. 13). This necessity arises from the preliminary analysis of data from germline samples, which revealed that copy number distributions can vary significantly between chromosomal arms, with some regions demonstrating stable values and others exhibiting subtle fluctuations (Fig. 14).

To establish these robust and standardised thresholds for identifying significant copy number alterations in 50kb genomic segments within each chromosome arm, the GWIS employed the Tukey outlier formula. This widely used statistical method determines outliers based on the interquartile range (IQR), which quantifies the spread between the 25th and 75th percentiles of the data. Specifically, the Tukey method sets thresholds at the median ± 1.5 times the IQR to identify moderate outliers (Fig. 13). Therefore, any genomic segment in a sample that exceeded the established threshold for its respective chromosomal arm, as determined using the healthy control samples, was deemed to have undergone a significant alteration (Fig. 15). The GWIS was subsequently computed by tallying the number of these significantly altered segments and expressing the total as a percentage of all 50kb segments across the genome. This calculation offers a measure of genomic instability using sWGS data derived from microdissected samples, providing insights into potential genomic instability in these specimens.

The GWIS measures overall genomic instability in our esophageal specimens. Given that genomic instability is a hallmark of many cancer types, not just oesophageal squamous cell carcinoma (OSCC), GWIS could be useful for other cancer diagnostics. Our results showed that GWIS captures genomic instability accurately, demonstrating a significant increase between neoplasia/dysplasia and normal/benign condition cytosponge samples. Notably, it was still significantly increased in dysplasia/neoplasia scenarios compared to benign conditions, indicating high specificity of this metric.

### Study objectives

The objectives of the study were 3-fold:
- Objective 1 (Sponge Samples Data) : Assess the Feasibility of Identifying Key Copy-Number-Related biomarkers in Non-Microdissected, Low-Cellularity Sponge Samples for Predicting Esophageal Squamous Dysplasia and Cancer through Machine Learning.
   The primary objective of this project was to determine the feasibility of identifying key copy number alteration (CNA)-related biomarkers in non-microdissected, low-cellularity Cytosponge samples for predicting oesophageal squamous dysplasia and cancer using machine learning techniques. Specifically, this study investigated 39 chromosome arms, utilising aggregated log2-transformed copy number values, along with a genome-wide instability score (GWIS). This study aimed to identify the most informative CNA biomarkers that could potentially serve as predictors for detecting these clinically significant conditions. Using the generalised linear model (GLM) implemented in the R package glmnet (Friedman et al., 2010), the relationship between the variable measures (39 chromosome arms and GWIS) and the presence of oesophageal squamous dysplasia and cancer was analysed. This machine learning method does not require prior knowledge of the outcome drivers. The focus was to accurately classify patients based on the endoscopy findings and identify the most significant features for this classification.
- Objective 2 (Sponge Samples Data): Evaluate the Predictive Accuracy of Genomic Features Identified in Aim 2, Combined with p53 Immunohistochemistry (IHC) and Squamous Cell Atypia, in Sponge Samples Using Logistic Regression.
   Building on the first objective, the second objective was to evaluate the predictive accuracy of the most informative CNV-related genomic features identified through the initial analysis. This evaluation combined these genomic features with cytology/pathology data (such as p53 immunohistochemistry and definite squamous cell atypia) and clinical data (including age, sex, BMI, and history of head and neck cancer) using simple logistic regression modelling.

### Example 1: Identifying key copy number related biomarkers in non-micro dissected low cellularity sponge samples for predicting oesophageal squamous dysplasia and cancer through machine learning

This study identified the most informative CNA biomarkers to serve as predictors for oesophageal squamous dysplasia and cancer.

### Data preprocessing

The dataset, comprising 242 samples from 178 Cytosponge tissue blocks obtained from the EDEN and OSCAR studies, consisted of 39 chromosome arm features, represented by their aggregated log2 copy number values, along with a genome-wide instability score. The outcome variable was binarised to indicate the presence or absence of oesophageal squamous dysplasia or neoplasia based on endoscopy findings. The use of 39 chromosomal arms (as listed in Figure 3) rather than arms from all 46 chromosomes is that certain regions can be excluded (those prone to technical artifacts, e.g. commonly found in peri-centromeric and sub-telomeric areas, through a blacklist developed from normal genome data. Therefore, these areas with known issues like repeat elements, low mappability regions can be excluded. The software also excludes sex chromosomes due to their complexity. The Y chromosome, in particular, has large repeat regions and low complexity sequences, leading to higher artifact rates. The variability of the X chromosome due to X-inactivation adds further complexity, making accurate CNA detection challenging. In summary, QDNAseq's optimized settings were used, automatically excluding problematic regions from the output.

### Model

Using the generalised linear model (GLM) implemented in the R package glmnet, the relationship between the variable measures (39 chromosome arms and GWIS) and the presence of oesophageal squamous dysplasia and cancer was analysed. This machine learning method does not require prior knowledge of the outcome drivers. The focus was to accurately classify patients based on the endoscopy findings and identify the most significant features for this classification.

### Elastic net model development

Penalised logistic regression with elastic net regularisation, which integrates the advantages of ridge and lasso regression, was implemented. Ridge regression addresses multicollinearity by reducing the magnitude of coefficients while retaining all predictors, while lasso regression improves interpretability by setting some coefficients to zero, thereby eliminating irrelevant features. Elastic net regularisation balances these two approaches by utilising the tuning parameter alpha (a), which ranges from 0 to 1 and controls the magnitude of the penalty term and regularisation strength parameter lambda (λ). This method enhances predictive performance, mitigates overfitting and multicollinearity issues, and improves model interpretability.

To optimise the hyperparameters, a stratified k-fold cross-validation approach was used to evaluate the performance of the elastic net logistic regression model across a range of alpha values. For each alpha value, 5-fold cross-validation was repeated ten times. This approach provides a reliable and robust assessment of the model's performance.

The cv.glmnet function from the glmnet package trained the model by selecting the optimal lambda value (lambda.min) that minimised the cross-validated mean squared error. To reduce overfitting, lambda. 1se, the largest lambda within one standard error of the minimum error, was selected. Model performance was evaluated by calculating the accuracy on the validation set, and the number of non-zero coefficients was recorded to assess the sparsity and feature selection properties of the model. The results for each alpha value were summarised across runs by calculating the mean and standard deviation of the accuracy and number of non-zero coefficients, providing a comprehensive view of the model's performance and sparsity.

Cross-validation results showed a consistent mean accuracy across different alpha values, ranging from 0.890 to 0.895, with the highest accuracy achieved at alpha levels of 0.1 and 0.4 (approximately 0.8949). This indicates that the mean classification accuracy remains consistently high (approximately 0.89) across all alpha values, suggesting that regularisation intensity has little impact on model accuracy in this scenario. Nevertheless, as the alpha values increased beyond 0.4, the accuracy decreased slightly, indicating that very high levels of regularisation had a negative impact on performance. The model sparsity also increased with higher alpha values, with all 40 coefficients being non-zero at alpha level 0 (ridge regression) and approximately 13.62 non-zero coefficients at alpha level 1 (Lasso regression).The standard deviation of accuracy was low across all alpha values, whereas the standard deviation of non-zero coefficients increased with higher alpha values, indicating greater variability in sparsity at higher regularisation strengths. An alpha value of 0.4 achieved a balance between high accuracy and model sparsity. This value resulted in a parsimonious model with fewer non-zero coefficients (mean: 19.76) compared to the alpha value of 0.1 (mean: 30.08), enhancing interpretability and simplicity.

To further validate the findings from the cross-validation analysis, the misclassification error and binomial deviance plots were examined for each alpha value. For alpha values between 0.1 and 0.4, the misclassification error plots showed a broader range of lambda values where the error remained low and stable. This suggests that models with these alpha values are less sensitive to the specific choice of lambda and are likely to generalise better to new data. Similarly, lower alpha values (0.1 to 0.4) exhibited a more gradual increase in binomial deviance, an indicator of model fit, indicating robust performance across a range of lambda values. This observation further strengthened the selection of alpha = 0.4 as the optimal value, as it minimised errors and binomial deviance while maintaining stability across various lambda values, thereby ensuring robustness. This choice effectively mitigated the risks of overfitting (when alpha values were low) and underfitting (when alpha values were high).

To determine the optimal lambda for the chosen alpha value, a composite score was calculated for each cross-validation run. This composite score balances the classification accuracy and model sparsity, measured by the proportion of non-zero coefficients. The lambda.1se, with the highest composite score across all cross-validation runs, was selected as the optimal lambda. This approach ensured that the chosen lambda provided the best trade-off between predictive performance and model interpretability, as sparser models with fewer non-zero coefficients are generally more interpretable and less prone to overfitting. The final model's coefficients were subsequently obtained by training the penalised logistic regression model on the entire dataset using the identified optimal alpha and lambda values.

### Leave one out analysis (LOO)

The performance and predictive capabilities of the developed model were evaluated using a leave-one-out (LOO) analysis approach. This involved iteratively training the penalised logistic regression model on all samples except one and then predicting the outcome for the excluded sample. This process was repeated for each sample in the dataset to ensure a comprehensive assessment. During the earlier k-fold cross-validation phase, the optimal hyperparameters were identified. The LOO analysis was then performed on the entire dataset using the 'glmnet' function with these specified hyperparameters. In each iteration, the excluded sample was treated as the test set for prediction. Predictive probability scores and non-zero coefficients contributing to the prediction were recorded.

Upon completion of the LOO analysis, the predictive probability scores were compiled, and a Receiver Operating Characteristic (ROC) curve was generated to assess the model's ability to distinguish between positive (dysplasia/neoplasia) and negative (non-dysplasia/neoplasia) cases. The Area Under the Curve (AUC) quantified the model's discriminative power, and Youden's J statistic determined the optimal prediction probability threshold for binarizing the scores. The predictive performance of the model was further evaluated using key metrics, such as sensitivity, specificity, and their corresponding 95% confidence intervals, derived from the LOO predictions.

### Predictor selection and refinement

An elastic net regression model was employed to identify key copy number alteration (CNA)-related features in non-microdissected, low-cellularity Cytosponge samples associated with dysplasia or neoplasia via endoscopy. This approach was chosen because of its ability to handle multicollinearity and perform variable selection simultaneously by combining the properties of both LASSO and ridge regression. Initially, the elastic net logistic regression model identified 17 of the 40 coefficients as significant predictors when using the optimal alpha and lambda parameters determined through the cross-validation process detailed in the previous sections. Following this initial selection, a more detailed analysis was conducted to identify the features with the strongest influence. This step was crucial for selecting predictors for downstream binomial logistic regression.

Drawing on the results of the previously conducted leave-one-out (LOO) analysis, the non-zero coefficients from each iteration were aggregated, and the percentage of samples with non-zero coefficients was calculated for each predictor. This iterative process identified predictors that consistently maintained their significance, thereby ensuring stability and reliability. A threshold of 70% was used to select the most robust predictors.

To assess the variability and potential impact of these robust predictors, the coefficient of variation of relative risks (cvRR) was calculated for each feature. The cvRR quantifies the variability of relative risks by dividing the standard deviation of the exponential relative risk values by the mean, with a higher cvRR indicating greater variability in the relative risks described by that predictor, and thus, a more substantial potential impact on the cohort.

A volcano plot was generated, plotting the cvRR against the magnitude of the regression coefficient, providing visual insights into the most critical predictors. To further quantify this effect, both metrics were normalised to a 0-1 range, and a composite score was calculated for each predictor by averaging these normalised values. The composite score captured both the variability (cvRR) and strength (coefficient magnitude) of each feature, with predictors having higher scores indicating a strong association with the outcome and significant variability, thereby highlighting their potential influence.

To determine the final set of predictors for inclusion in the logistic regression model, a quantile threshold of 0.75 (top 25%) for the composite score was employed. This rigorous selection process ensured that the final logistic regression model was built on the most robust and impactful predictors, thereby enhancing the predictive accuracy and reliability of the model.

### Results:

The results are shown in Figures 2-6.

We first evaluated the robustness of each predictor across different LOO models. This process identified predictors that consistently maintained their significance across various iterations of the model, thereby ensuring their stability and reliability. This analysis provided insight into the magnitude and consistency of each predictor's effect. By overlaying these two methods-magnitude across LOO iterations and cvRR-we were able to discern the strongest predictors. A composite score that factors in both metrices were used in narrowing down.

The initial exploratory analysis focused on detecting copy number alterations (CNAs) across the genome by examining relative log2 copy number values measured for multiple 50-kilobase genomic segments spanning the 39 autosomal chromosome arms (as listed in Fig 3 dotchart). Descriptive statistics revealed minimal CNAs in participants with normal endoscopic findings and those with benign conditions, as evidenced by their comparable median log2 values of -0.005 (IQR: [-0.016, 0.001]) and -0.005 (IQR: [-0.015, 0.001]), respectively. In contrast, the dysplasia/neoplasia group displayed a slightly higher median log2 value of -0.0045 (IQR: [-0.019, 0.004]), suggesting the presence of more pronounced CNAs.

To determine whether there were statistically significant differences in CNAs between the endoscopic outcome groups, the Kruskal-Wallis test was performed, yielding a p-value of less than 0.0001. To further explore these overall differences, pairwise comparisons were performed using the Wilcoxon rank-sum test with Hochberg adjustment for multiple comparisons. The results showed significant differences between all pairs of groups: benign vs. dysplasia/neoplasia (p < 0.0001), benign vs. normal (p < 0.0001), and dysplasia/neoplasia vs. normal (p < 0.0001). However, the effect sizes measured by Cliffs delta were small, varying between 0.0156 and 0.0214, indicating that, although differences in CNAs were detectable, their magnitude was modest. This was expected, considering the issue of low cellularity caused by normal tissue contamination in Cytosponge samples.

Figure 2 displays log2 - transformed relative copy number values for individual Cytosponge samples across chromosomal arms 1p to 22q, divided into three panels based on endoscopic outcomes: normal (n = 116), benign conditions (n = 53), and dysplasia/neoplasia (n = 73). The vertical axis represents individual samples, whereas the horizontal axis lists the chromosomal arms. Each bubble represents a sample, with the bubble size proportional to the magnitude of the log2 copy number change, and the colour indicating the direction and extent of the alteration: blue for deletions (negative log2 values), red for amplifications (positive log2 values), and gray for no significant change (near zero log2 values). The plot reveals increased variability and prevalence of copy number alterations across specific chromosomal arms in the dysplasia/neoplasia group compared to the normal and benign condition groups.

Figure 3 scatter plots display log2 transformed copy number values for individual Cytosponge samples across chromosomal arms 1p to 22q, categorised by endoscopic outcomes: normal (n = 116), benign conditions (n = 53), and dysplasia/neoplasia (n = 73). Dots represent individual data points, colour-coded by the study participant group: healthy control (green), high-risk (blue), and dysplasia/neoplasia (red). The lower bar charts show the sample counts for each study participant group within the corresponding endoscopic outcome category. The vertical spread of data points indicates variability in copy number alterations across the genome. Cytosponge samples from individuals with dysplasia/neoplasia exhibit higher variability in copy number alterations compared to normal and benign conditions, reflecting increased genomic instability associated with these conditions.

To further refine the analysis and gain a more granular understanding of the genomic alterations, we examined CNAs at the chromosomal arm level, comparing Cytosponge samples collected from participants with dysplasia/neoplasia to those collected from participants without these conditions (benign and normal endoscopic findings combined) (Fig.2 bubble plot). The Wilcoxon rank-sum test was applied to each chromosomal arm, and the results were adjusted for multiple comparisons using the stringent Hochberg method.

Notably, chromosomal arms 3q, 9q, 9p, 4p, 7q, 20q, 11p, 11q, 2q, 2p, and 1q exhibited highly significant differences, with adjusted p-values ranging from 3.90E-11 to 2.45E-03 and moderate effect sizes ranging from 0.304 for 1q to 0.458 for 3q. These findings suggest that these specific genomic regions are particularly susceptible to copy number changes in participants with dysplasia or neoplasia and that the observed differences are of considerable practical relevance. Furthermore, several other chromosomal arms, including 16q, 3p, 10p, 17q, 21q, 16p, 7p, 4q, 5q, 17p, 12q, 13q, 8p, and 8q, also exhibited statistically significant differences in CNAs between the two groups, with adjusted p-values ranging from 1.41E-03 to 8.61E-03. However, the effect sizes for these arms were generally smaller, ranging from 0.180 for 8q to 0.260 for 16q, suggesting a modest magnitude of difference. Interestingly, a subset of chromosomal arms, including 6p, 18q, 18p, 10q, 12p, 14q, 15q, 19p, 19q, 1p, 22q, 5p, and 6q, did not exhibit statistically significant differences in their CNAs after correcting for multiple comparisons.

Arm-level CNA analysis showed a complex genomic landscape in participants with dysplasia/neoplasia, with significant alterations in specific chromosomal regions. The identification of chromosomal arms with statistical significance and moderate or small effect sizes suggests that these regions may play a role in dysplasia and neoplasia development and progression with varying degrees of impact. This detailed view of genomic alterations provides valuable insights into the molecular mechanisms underlying these conditions and may inform targeted therapeutic strategies.

Figure 4 shows the distribution of GWIS values for each endoscopic outcome group: normal (n = 116), benign conditions (n = 53), and dysplasia/neoplasia (n = 73). Individual data points are overlaid and coloured by study participant risk groups: healthy control (green), high-risk (blue), and dysplasia/neoplasia (red). Significant differences in GWIS between groups are indicated by asterisks, with the number of asterisks representing the level of significance based on pairwise Wilcoxon tests with Hochberg correction for multiple comparisons (**** p < 0.0001). The dysplasia/neoplasia group exhibited substantially higher GWIS compared to the normal and benign conditions groups, reflecting increased genomic instability associated with these conditions.

To capture the extent of genomic instability more comprehensively, the Genome-Wide Instability Score (GWIS) was introduced as a complementary metric (Fig 4: boxplot). Descriptive statistics for GWIS revealed substantially higher genomic instability in the dysplasia/neoplasia group (median = 0.363, IQR: 0.364) than in the benign condition (median = 0.0638, IQR: 0.103) and normal groups (median = 0.0653, IQR: 0.151).

In contrast to the CNA analysis, where all pairwise comparisons were statistically significant, the GWIS analysis showed highly significant differences only between the benign conditions and dysplasia/neoplasia groups (adjusted p-value = 8.66e-11) and between the dysplasia/neoplasia and normal groups (adjusted p-value = 3.63e-13). Notably, the effect sizes for these comparisons, calculated using Cliff's delta, were large, with 0.588 for benign conditions vs. dysplasia/neoplasia and 0.540 for dysplasia/neoplasia vs. normal conditions, indicating substantial differences in genomic instability between these groups.

Interestingly, no significant difference in GWIS was observed between the benign and normal groups (adjusted p = 0.766), with a small effect size of 0.0231. This finding contrasts with that of the preceding CNA exploratory analysis, in which a significant difference was detected between the two groups, albeit with a small effect size. These findings suggest that while the benign conditions and normal groups exhibited similar levels of genomic instability, the dysplasia/neoplasia group displayed substantially higher genomic instability, as evidenced by the large effect sizes. This pattern aligns with the expectation that individuals with dysplasia or neoplasia will exhibit greater genomic instability than those with benign conditions or normal findings.

As can be seen in Figure 6, significant indicators of esophageal squamous dysplasia and cancer within sponge specimens encompass alterations in copy number across chr2q, chr3q, chr9p, chr11q alongside the Genome Wide Instability Score (GWIS). The identification of 2q gain as a significant predictor of dysplasia and neoplasia is novel.

Our analysis of CNAs in Cytosponge samples provides valuable insights into the progression of oesophageal squamous cell carcinoma (OSCC) and demonstrates the potential of CNAs as biomarkers for early detection. Significant differences in CNAs (copy number alteration) at both the arm level and genome-wide scales were observed across various endoscopic outcome groups, despite the challenge of low cellularity in Cytosponge samples. These findings suggest that CNAs associated with dysplasia and neoplasia can be detected even with normal tissue contamination.

### Example 2: The predictive model is accurate for detecting OSCD and OSCC

Multiple predictive models were developed and assessed incorporating various combinations of genomic, pathology/cytology, and clinical features. This approach utilized simple logistic regression models to predict oesophageal squamous dysplasia/neoplasia. For training each model the outcome variable was binarised to indicate the presence or absence of oesophageal squamous dysplasia or neoplasia based on endoscopy findings.

### Clinical data model

The clinical data model included data such as age, sex, body mass index (BMI), previous chest radiotherapy and history of head and neck cancer. These variables were binarized as yes or no.

### Copy-number information model

The predictive accuracy of the features obtained in example 1 were assessed, referred to below as the 'copy number information model'. This was initiated with univariate logistic regression on each genomic feature identified to be in the top 25% quantile by composite score from previous analysis (chr2q , chr3q, chr9p, chr11q, genome wide instability score (GWIS)) as indicated in Figure 6. Only features that exhibited statistical significance via univariate analysis and a subsequent stepwise selection process based on AIC (Akaike Information Criterion) and BIC (Bayesian Information Criterion) criteria were then incorporated into the final copy number information model.

### Sponge pathology model

This model binarized the variables of the pathology report, with atypical squamous cells of undetermined significance (AUS), low-grade dysplasia (LGD), high-grade dysplasia (HGD), and oesophageal squamous cell carcinoma (OSCC) labelled as 1, while normal findings or inflammatory conditions such as oesophagitis were marked as 0. For p53 IHC, positive or equivocal staining was assigned a value of 1, whereas negative staining was marked as 0.

### Integrated models

Combined models integrating two or all three data types were also constructed. For each model, stepwise selection based on the Akaike Information Criterion (AIC) was employed to identify the most significant predictors. This iterative process involved adding or removing variables to minimize the AIC, balancing model fit and complexity. The performance of each model was evaluated using metrics such as the Area Under the Receiver Operating Characteristic Curve (AUROC), sensitivity, specificity, accuracy, and the Youden Index. Receiver Operating Characteristic (ROC) curves were generated to compare the performance of the different models, depicting the trade-off between the true positive rate (sensitivity) and the false positive rate (1 - specificity) at various classification thresholds. These curves allowed for visual comparison and assessment of the models' discriminative abilities.

Predictors with p-values ≤ 0.05 were considered significant and included in the stepwise selection process. Stepwise selection was conducted using the stepAIC function from the MASS package in R software, optimising for the lowest AIC value. This comprehensive approach ensured that the most relevant predictors were identified and that the models developed were robust and reliable for predicting oesophageal squamous dysplasia/neoplasia.

### Results:

The diagnostic performance of the tested models is shown in Table 1.

**Table 1:**

| **Model Type** | **AUROC** | **Sensitivity** | **Specificity** | **Youden Threshold** |
|---|---|---|---|---|
| Clinical data (age, previous head and neck cancer) | 0.609 [0.536, 0.610] | 0.904 | 0.361 | 0.265 |
| Sponge pathology data (sponge definite squamous atypia [AUS, LGD, HGD, or OSCC], p53 IHC) | 0.868 [0.817, 0.870] | 0.836 | 0.846 | 0.682 |
| Copy number data (chr2q, chr3q, chr9p, chr11q, GWIS) | 0.947 [0.917, 0.948] | 0.89 | 0.882 | 0.772 |
| Sponge pathology & clinical data (sponge definite squamous atypia [AUS, LGD, HGD, or OSCC], p53 IHC, Previous chest radiotherapy) | 0.880 [0.829, 0.882] | 0.836 | 0.846 | 0.682 |
| Copy number & Sponge pathology data (sponge definite squamous atypia [AUS, LGD, HGD, or OSCC], p53 IHC, chr2q, chr3q, chr9p, GWIS) | 0.969 [0.942, 0.971] | 0.959 | 0.905 | 0.864 |

The model combining the copy number model features and sponge pathology features shows increased diagnostic accuracy compared to the accuracy of the single modality models. The ROC curve for comparative model performance can be seen in Figure 7. Among logistic regression models incorporating clinical, pathology, and genomic copy number data, the combination of pathology and genomic data yielded the highest AUROC (0.969), with genomic data models slightly outperforming pathology data models, emphasizing the value of genomic copy number information in prediction accuracy.

The model was also run on the copy number model alone without including GWIS as an input variable. The AUROC was 0.939, sensitivity was 0.849 and specificity was 0.911.

### Example 3: Validation of the model on microdissected endoscopic therapy samples

### Participants

The copy number model was validated in a different sample with 32 patient cases and 110 samples sequenced as shown in Figure 8a. The participants were adult patients who previously underwent endoscopic treatment including endoscopic submucosal dissection for oesophageal squamous dysplasia or neoplasia (LGD, HGD, and early OSCC). The samples were multi-region endoscopic submucosal dissection specimens.

Each of the 130 archival tissue blocks selected for this study underwent meticulous pathological re-evaluation to confirm the accuracy of prior diagnoses and the adequacy of cellular material for detailed molecular analysis (see Figure 8b). Upon verifying sufficient cellularity, the tissue blocks were systematically processed, involving the cutting of multiple 4µm haematoxylin and eosin (H&E) stained slides and interspersed 10µm thick blank slides. Two expert gastrointestinal pathologists carefully reviewed the H&E-stained slides. These annotated slides served as maps for identifying and marking the regions of interest, which are essential for the subsequent microdissection process. This approach ensured that the areas selected for microdissection accurately represented the desired disease grades. Additionally, the surrounding connective tissue was microdissected and utilised as a reference germline (negative control) instead of unavailable blood samples because of the archival nature of the samples.

To optimise the DNA yield, additional sectioning and microdissection were performed as necessary, considering the distribution of disease grades within the tissue blocks. Prior to microdissection, blank sections were de-paraffinised and dehydrated. The microdissection itself was conducted under a microscope using a 21G needle, guided by the previously marked H&E sections. During microdissection, the collected lesions were preserved in 70% ethanol until all tissue sections were microdissected. Subsequently, the samples were vortexed, ethanol was removed, and the DNA was extracted.

Figure 8a shows a chart illustrating the distribution of 110 DNA samples collected from 130 microdissected endoscopic ESD tissue blocks across various pathological conditions in 32 patient cases. The segments of the chart represent the number of DNA samples corresponding to each pathology.

### Data

Data was collected and pre-processed as discussed above.

### Results

The results can be seen in Figures 8-11.

To provide additional support for this analysis, a graphical representation was used to illustrate the progression of genomic instability across different pathological stages, ranging from ANE to OSCC (Fig. 10). This plot, derived from normalised data, depicts the changes in log2-transformed relative copy number values across chromosomal arms for various specimen pathologies. These results indicate that CNAs increased progressively from ANE to OSCC. The ANE and LGD samples exhibited relatively low levels of CNAs, suggesting early genomic stability. However, a substantial increase in CNAs was observed between the LGD and HGD stages, suggesting that significant genomic alterations may be critical for the transition from the precancerous stages to invasive carcinoma.

Figure 11 shows the progressive increase in GWIS from ANE to LGD to HGD and OSCC, as illustrated in the boxplot which validates GWIS as a reliable metric for quantifying genomic instability and stratifying OSCC disease progression. This harmony between GWIS values and the extent of CNAs underscores the clinical significance of genomic instability metrics in guiding diagnosis and treatment decisions for OSCC.

In addition to the genome-wide instability typically observed in the progression of oesophageal squamous cell carcinoma (OSCC), distinct patterns of copy number alterations (CNAs) were observed across specific chromosome arms (Fig 9).

As can be seen from Figure 12, the genomic features associated with disease state that were identified in sponge sample data were recapitulated in the ESD data. The copy number model as described in Example 2 was used to predict on the ESD microdissected samples. The results of the analyses are shown in Tables 2 and 3.

**Table 2:**

| **Sample** | **AUROC** | **Sensitivity** | **Specificity** | **Youden threshold** |
|---|---|---|---|---|
| Sponge sample | 0.947 | 0.89 | 0.882 | 0.772 |

**Table 3:**

| **Test data configuration (micro dissected samples)** | **AUROC** | **Sensitivity** | **Specificity** | **Youden threshold** |
|---|---|---|---|---|
| LGD treated as '1' | 0.879 | 0.8226 | 0.8958 | 0.985 |
| Excluding LGD | 0.905 | 0.889 | 0.8958 | 0.989 |

Significant indicators of oesophageal squamous dysplasia and cancer within sponge specimens encompass alterations in copy number across chr2q, chr3q, chr9p chr11q, alongside the Genome Wide Instability Score (GWIS). Utilizing these markers, a straightforward logistic regression model demonstrated robust efficacy when also tested on microdissected samples. The model was evaluated with and without LGD to demonstrate the pathological variability within the LGD group. Removing LGD improves the model performance.

LGDs , analogous to healthy tissues, typically exhibit minimal malignant potential and rarely gain a competitive advantage at both genomic and tissue levels. While a small subset of LGDs may manifest genomic alterations associated with malignant progression, they often present phenotypically as LGDs. This understanding is reinforced in follow-ups: most LGDs did not progress to OSCC, except for a small percentage . However, clinically categorizing both LGDs and HGDs as dysplasia can induce significant psychological distress in patients and impose a burden on clinical screening processes. Therefore, there's a compelling need to identify high-risk dysplasia at the genomic level to enhance pathological diagnosis and guide clinical management effectively. Moreover, the pathological features of LGDs aren't clear-cut and introduce a level of observed disagreement among pathologists. Thus, the model was tested both with and without LGD to demonstrate the pathological variability within the LGD group, as noted in the literature, and to show that removing LGD from the model improves its performance, meaning that the model is able to detect HGD and OSCC better than LGD (and this fitting with what is needed in the clinic).

## Claims

1. A computer-implemented method for detecting oesophageal squamous cell dysplasia (OSCD) or oesophageal squamous cell cancer (OSCC) in a cell sample from a subject, the method comprising:
a) obtaining the copy number variation (CNV) in the cell sample across chromosomal arms comprising: 2q, 3q, 9p and 11q;
b) inputting the CNV from step a) into a trained machine learning model which relates CNV across the chromosomal arms comprising 2p, 3q, 9p and 11q with OSCD or OSCC; and
c) outputting from the trained model a prediction comprising the presence or absence of OSCD or OSCC.

2. The method of claim 1, further comprising:
a) obtaining a genome wide instability score (GWIS) for the cell sample, wherein the genome wide instability score is a measure of the CNV across a plurality of autosomal arms in the cell sample; and
b) inputting the GWIS into the trained model wherein the GWIS is an additional input variable of the trained model.

3. The method of any one of the previous claims wherein the cell sample is a non-endoscopic sample.

4. The method of claim 3, further comprising:
a) obtaining:
i) a cytological evaluation; and/or
ii) immunohistochemistry data,
for the subject, and
b) inputting i) and/or ii) into the trained model, wherein the cytological evaluation and/or immunohistochemistry data is(are) an additional input variable(s) of the trained model,
optionally, wherein:
iii) the cytological evaluation comprises categorising one or more cells as any of: atypical squamous cells of undetermined significance (AUS), low-grade dysplasia (LGD), high-grade dysplasia (HGD), oesophageal squamous cell carcinoma (OSCC) or normal squamous oesophagus; and/or
iv) the immunohistochemistry data is obtained from testing one or more cells for the presence of a cancer biomarker, optionally wherein the biomarker is p53.

5. A computer-implemented method for generating a trained model to detect OSCD or OSCC, the method comprising:
a) obtaining measurements for the CNV across chromosomal arms in cell samples from subjects, the chromosomal arms comprising: 2q, 3q, 9p, and 11q wherein the cell samples are from subjects who have OSCD or OSCC;
b) obtaining measurements for the CNV across the chromosomal arms comprising: 2q, 3q, 9p, and 11q in cell samples from subjects who do not have OSCD or OSCC; and
c) training the model to relate the CNV measurements with the presence or absence of OSCD or OSCC to generate a trained model.

6. The method of claim 5, wherein the trained model is trained on an additional input variable: a genome wide instability score (GWIS), wherein the genome wide instability score is a measure of the CNV across a plurality of autosomal arms in the cell sample, the method further comprising obtaining measurements of the GWIS from the cell samples and training the model to relate CNV and GWIS of the cell samples with the presence or absence of OSCD or OSCC.

7. The methods of any of claims 5-6 wherein the cell samples are non-endoscopic samples.

8. The method of claims 7, wherein the trained model is trained on one or more additional input variable(s):
a) cytological evaluation; and/or
b) immunohistochemistry data,
the method further comprising obtaining a) and/or b) for the cell samples and training the model to relate CNV and cytological evaluation and/or immunohistochemistry data of the cell samples with the presence or absence of OSCD or OSCC,
optionally wherein:
i) the cytological evaluation comprises categorising one or more cells as any of: atypical squamous cells of undetermined significance (AUS), low-grade dysplasia (LGD), high-grade dysplasia (HGD), oesophageal squamous cell carcinoma (OSCC) or normal squamous oesophagus; and/or
ii) the immunohistochemistry data is obtained from testing one or more cells for the presence of a cancer biomarker, optionally wherein the biomarker is p53.

9. A computer-implemented trained model obtained from the method of any of claims 5-8.

10. A computer-readable storage medium or a computer program comprising instructions which, when executed by a computer, cause the computer to carry out the methods according to any of claims 1-4.

11. A kit comprising the computer program or computer-readable storage medium of claim 11 and a non-endoscopic cell collection device.

12. The methods of any of claims 1-8, wherein the CNV:
is measured from shallow whole genome sequencing (sWGS) data from the cell sample.

13. An apparatus comprising:
a) a memory storing a trained machine learning model; and
b) processing circuitry communicatively coupled to the memory, the processing circuitry to run the trained machine learning model to receive as input CNV measurements from a cell sample from a subject, wherein the CNV measurements are across chromosomal arms comprising: 2q, 3q, 9p, and 11q; and
output a prediction corresponding to the presence of absence of OSCD or OSCC.

14. An apparatus comprising:
a) a memory storing a machine learning algorithm; and
b) processing circuitry communicatively coupled to the memory, the processing circuitry to run the machine learning algorithm to process training data to generate a trained machine learning model by
adjusting the parameter values of the machine learning algorithm to reduce an error function based on training data comprising CNV measurements and associated ground truth data,
wherein the CNV measurements are across chromosomal arms comprising: 2q, 3q, 9p, and 11q in cell samples from each of a plurality of subjects;
and wherein the associated ground truth data comprises, for each of the plurality of training subjects an indication of the presence or absence of OSCD or OSCC.

15. Use of GWIS as a diagnostic marker for cancer, wherein GWIS is a measure of the CNV across a plurality of autosomal arms in a cell sample from a subject.
